(19) Europäisches Patentamt
European Patent Office
Office européen des brevets

(11) Numéro de publication : **0 172 058 B1**

(12) # FASCICULE DE BREVET EUROPÉEN

(45) Date de publication du fascicule du brevet : 03.01.90

(21) Numéro de dépôt : 85401342.2

(22) Date de dépôt : 03.07.85

(51) Int. Cl.$^5$ : **C 07 D471/04**, A 61 K 31/435 // (C07D471/04, 221:00, 221:00)

(54) **Nouvelles phényl-naphthyridines, leur procédé de préparation, médicaments les contenant, notamment anti-ulcères.**

(30) Priorité : 11.07.84 FR 8411040

(43) Date de publication de la demande : 19.02.86 Bulletin 86/08

(45) Mention de la délivrance du brevet : 03.01.90 Bulletin 90/01

(84) Etats contractants désignés : AT BE CH DE FR GB IT LI LU NL SE

(56) Documents cités :
EP-A- 0 000 490
EP-A- 0 018 735
US-A- 4 215 123

(73) Titulaire : LABORATOIRES UPSA
1 bis, rue du Docteur Camille Bru
F-47000 Agen (FR)

(72) Inventeur : Teulon, Jean-Marie
13, Avenue Guibert
F-78170 La Celle Saint Cloud (FR)

(74) Mandataire : Portal, Gérard et al
Cabinet Beau de Loménie 55, rue d'Amsterdam
F-75008 Paris (FR)

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 172 058 B1

Jouve, 18, rue St-Denis, 75001 Paris, France

## Description

La présente invention concerne les phényl-naphtyridines de formule (I). Elle concerne également le procédé de préparation desdits produits et leurs applications.

Dans le document EP-A-0 018 735, on décrit de nouveaux dérivés 4-amino et 4-amino substitué 1,6-naphtyridine, pouvant comporter divers substituants également en position 1, en position 3 ou en position 5, 6 ou 7 de la naphtyridine. Ces dérivés sont décrits comme ayant une activité anti-sécrétrice capable de réduire le volume gastrique total, la sécrétion en ions hydrogène ou la concentration en ions hydrogène, ce qui permet de traiter les ulcères peptiques.

De même, le document US-A-4 215 123 décrit des dérivés 4-oxy-3-carboxy ou cyano-1,2-dihydro-2-oxo-1,8-naphtyridine ayant la même activité anti-sécrétrice et permettant le traitement d'ulcères peptiques.

On connaît encore par le document ER-A-0 000 490 des naphtyridinones substituées ayant une activité d'inhibition des sécrétions gastriques dont l'atome d'azote en position 1 du cycle naphtyridinone est substitué par un radical alkylamino de formule

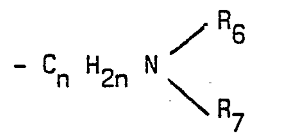

.

Les nouveaux composés selon l'invention sont choisis parmi l'ensemble constitué par les composés de formule (I) :

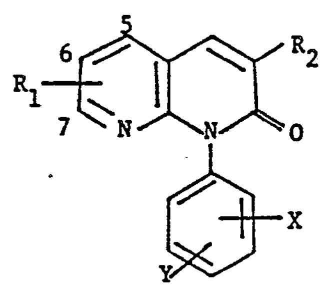

(I)

dans laquelle :

$R_1$ représente un atome d'hydrogène, un radical méthyle, isopropyle ou cyclopropyle, et peut être en position 5, 6 ou 7 de la naphtyridine,

$R_2$ représente un atome d'hydrogène, un radical alkyle inférieur linéaire ou ramifié de 1 à 5 atomes de carbone,

X et/ou Y représentent un atome d'hydrogène, un radical alkyle inférieur de 1 à 5 atomes de carbone, un atome d'halogène, un groupement trifluorométhyle, méthoxy, nitro, thiométhyle et peuvent être en position ortho, méta ou para.

Selon une variante de réalisation, $R_1$ est en position 7 de la naphtyridine et selon une autre variante, $R_1$ est H.

Selon une autre variante de réalisation, $R_2$ = H, $CH_3$ ou $C_2H_5$, de préférence $R_2 = CH_3$.

Egalement de préférence, X = m — $CF_3$ et Y = H.

Les composés de formule (I) selon l'invention peuvent être synthétisés :

— soit par une réaction de Réformatski suivie d'une déshydratation,
— soit par une réaction de Wittig suivie d'une cyclisation,
— soit par une réaction de Perkin,

sur un aldéhyde de formule (II) :

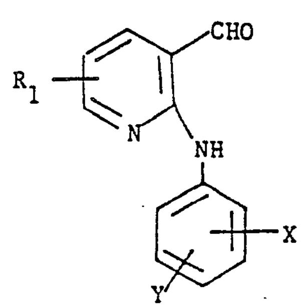

(II)

dans la formule (II), $R_1$, X et Y sont définis comme ci-dessus.

D'une façon générale, les aldéhydes de formule (II) peuvent être obtenus par oxydation, à l'aide d'un oxydant doux tel que par exemple $MnO_2$, dans un solvant organique comme le dichloro-méthane ou le chloroforme, à une température comprise entre 20 et 50 °C, d'un alcool de formule (III) :

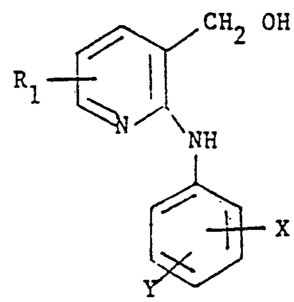

(III)

dans la formule (III), $R_1$, X et Y sont définis comme ci-dessus.

Les alcools de formule (III) sont obtenus par réduction à l'aide d'un réducteur classique tel que par exemple l'hydrure double d'aluminium et de lithium dans un solvant organique tel que par exemple le tétrahydrofuranne d'un acide ou d'un de ses esters de formule (IV) :

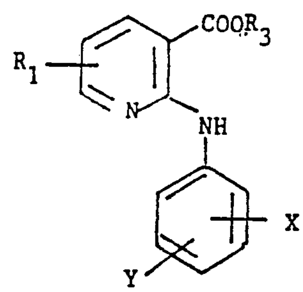

(IV)

dans la formule (IV), $R_1$, X et Y sont définis comme ci-dessus, $R_3$ est un atome d'hydrogène ou un alkyle inférieur de 1 à 5 atomes de carbone.

Le procédé préféré industriellement pour la synthèse des produits de formule (I) consiste dans l'utilisation de la réaction — connue — de Perkin par laquelle on fait réagir un aldéhyde de formule (II) sur un anhydride de formule (V)

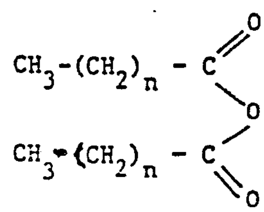

(V)

dans laquelle n est un nombre de 0 à 4 ou sur un sel de sodium de l'acide correspondant. La réaction peut avantageusement être réalisée dans un solvant tel que la méthylpyrrolydone à une température de 100 à 200 °C environ.

Selon l'invention, on propose des compositions thérapeutiques utiles notamment pour le traitement des ulcères gastro-intestinaux, caractérisées en ce qu'elles renferment, en association avec un excipient physiologiquement acceptable, au moins un composé de formule (I). Certains composés de formule (I) présentent en outre des propriétés anti-inflammatoires et analgésiques.

D'autres caractéristiques et avantages de l'invention seront mieux compris à la lecture qui va suivre de quelques exemples de préparation donnés à titre d'illustration.

Le tableau I ci-après donne la formule développée de certains produits.

Exemple 1

[(trifluorométhyl)-3 phényl] amino-2 hydroxy méthyl-3 pyridine

Formule III $R_1 = Y = H$ ; $X = m\text{-}CF_3$

A une suspension de 52 g d'hydrure double d'aluminium et de lithium dans 1000 ml d'éther éthylique anhydre, on ajoute goutte à goutte une solution de 200 g d'acide (trifluorométhyl-3 phényl amino)-2 nicotinique dans 500 ml de tétrahydrofuranne anhydre. Après la fin de l'addition, le mélange réactionnel est porté 3 h au reflux. Après refroidissement, l'excès d'hydrure double est détruit par addition d'acétate d'éthyle, puis d'une solution aqueuse saturée de sulfate de sodium. Le précipité formé est essoré et lavé à l'éther. Les filtrats rassemblés sont évaporés sous vide et on récupère 185,2 g de [(trifluorométhyl)-3 phényl] amino-2 hydroxy méthyl-3 pyridine sous forme de cristaux de point de fusion 103-105 °C.

Selon cette méthode sont préparés les dérivés suivants :

— méthyl-6 [(trifluorométhyl)-3 phényl] amino-2 hydroxy méthyl-3 pyridine

Formule III $R_1 = CH_3$ en 6 ; X = H ; X = m-$CF_3$

Cristaux F < 50 °C ; rendement 88 %

— isopropyl-6 [(trifluorométhyl)-3 phényl] amino-2 hydroxy méthyl-3 pyridine

Formule III $R_1$ = isopropyle en 6 ; Y = H ; X = m-$CF_3$

Huile ; rendement 93 %

— cyclopropyl-6 [(trifluorométhyl)-3 phényl] amino-2 hydroxy méthyl-3 pyridine

Formule III $R_1$ = cyclopropyle en 6 ; Y = H ; X = m-$CF_3$

Cristaux F = 90 °C ; rendement 92 %

— [(méthyl)-2 (chloro)-3 phényl] amino-2 hydroxy méthyl-3 pyridine

Formule III $R_1$ = H ; X = o-$CH_3$ ; Y = m-Cl

Cristaux F = 98-100 °C ; rendement 89 %

— phényl amino-2 hydroxy méthyl-3 pyridine

Formule III $R_1$ = X =Y = H

Huile ; rendement 95 %

Exemple 2

(trifluorométhyl-3 phényl amino)-2 nicotinaldéhyde

Formule II $R_1$ = Y = H ; X = m-$CF_3$

A une solution de 185 g de [(trifluorométhyl)-3 phényl] amino-2 hydroxy méthyl-3 pyridine, préparés à l'exemple 1 dans 2300 ml de chloroforme, on ajoute par petites fractions 690 g de $MnO_2$. Après la fin de l'addition, on agite à température ambiante durant 6 h. Le milieu réactionnel est ensuite filtré sur célite et le filtrat est évaporé à sec. Les cristaux obtenus alors (175 g) sont recristallisés dans l'heptane. On récupère ainsi 160 g de (trifluorométhyl-3 phénylamino)-2 nicotinaldéhyde sous forme de cristaux de point de fusion 80-81 °C.

Selon cette méthode sont préparés les dérivés suivants :

— méthyl-6 (trifluorométhyl-3 phénylamino)-2 nicotinaldéhyde

Formule II $R_1 = CH_3$ en 6 ; Y = H ; X = m-$CF_3$

Cristaux F = 52-54 °C ; rendement 95 %

— isopropyl-6 (trifluorométhyl-3 phénylamino)-2 nicotinaldéhyde

Formule II $R_1$ = isopropyle en 6 ; Y = H ; X = m-$CF_3$

Cristaux F < 50 °C ; rendement 92 %

— cyclopropyl-6(trifluorométhyl-3 phénylamino)-2 nicotinaldéhyde

Formule II  $R_1$ = cyclopropyle en 6 ; Y = H ; X = m-$CF_3$

Cristaux F = 82 °C ; rendement 94 %

— (méthyl-2 chloro-3 phénylamino)-2 nicotinaldéhyde

Formule II  $R_1$ = H ; X = o-$CH_3$ ; Y = m-Cl

Cristaux F = 115 °C ; rendement 88 %.

— phénylamino-2 nicotinaldéhyde

Formule II  $R_1$ = X = Y = H

Cristaux F = 77-8 °C ; rendement après recristallisation dans l'éther l'éther isopropylique 80 %.

Exemple 3

(Trifluorométhyl-3 phényl)-1 dihydro-1,2 oxo-2 méthyl-3 napthtyridine-1,8

Formule I  $R_1$ = Y = H ; X = m-$CF_3$ ; $R_2$ = $CH_3$

On prépare une solution de 50 g de (trifluorométhyl-3 phényl amino)-2 nicotinaldéhyde synthétisés à l'exemple 2, 28 ml de bromo-2 propionate d'éthyle dans 200 ml de benzène anhydre. 20 ml de cette solution sont versés sur 16 g de poudre de zinc activée. Par chauffage, la réaction de Réformatsky démarre, puis le reste de la solution est additionnée de telle façon que le reflux soit entretenu. Après la fin de l'addition, le reflux est maintenu durant 2 h.

Le mélange réactionnel est ensuite refroidi puis hydrolysé par de l'acide sulfurique dilué.

La phase organique séparée, les eaux mères sont extraites au chloroforme. Les phases organiques sont séchées sur sulfate de sodium, puis concentrées sous vide. Le résidu obtenu, 60 g, est repris par 400 ml de toluène et 20 ml de $POCl_3$, puis on porte au reflux durant 2 h. Après refroidissement, la phase organique est lavée à l'eau, puis séchée et concentrée sous vide. On obtient ainsi un résidu de 35 g qui cristallise. Après recristallisation dans l'isopropanol, on récupère 24 g de (trifluorométhyl-3 phényl)-1 dihydro-1,2 oxo-2 méthyl-3 naphtyridine-1,8 sous forme de cristaux de point de fusion 189-190 °C.

Exemple 4

(Trifluorométhyl-3 phényl)-1 dihydro-1,2 oxo-2 naphtyridine-1,8

Formule I  $R_1$ = $R_2$ = Y = H ; X = m-$CF_3$

Selon le mode opératoire de l'exemple 3, mais en utilisant 43,5 g de (trifluorométhyl-3 phényl amino)-2 nicotinaldéhyde préparés à l'exemple 2 et 23 ml de bromoacétate d'éthyle, on obtient après recristallisation dans l'isopropanol 15 g de (trifluorométhyl-3 phényl)-1 dihydro-1,2 oxo-2 naphtyridine-1,8 sous forme de cristaux de point de fusion 159-160 °C.

Exemple 5

(Trifluorométhyl-3 phényl)-1 dihydro-1,2 oxo-2 éthyl-3 naphty-1,8

Formule I  $R_1$ = Y = H ; X = m-$CF_3$ ; $R_2$ = $C_2H_5$

Selon le mode opératoire de l'exemple 3 mais en utilisant 30 g de (trifluorométhyl-3 phényl amino)-2 nicotinaldéhyde préparés à l'exemple 2 et 19,2 ml de bromo-2 butyrate d'éthyle on obtient après recristallisation dans l'isopropanol 8 g de (trifluorométhyl-3 phényl)-1 dihydro-1,2 oxo-2 éthyl-3 naphtyridine-1,8 sous forme de cristaux de point de fusion 144-146 °C.

Toujours selon cette méthode sont préparés les dérivés suivants :

Exemple 6

(Trifluorométhyl-3 phényl)-1 dihydro-1,2 oxo-2 diméthyl-3,7 naphtyridine-1,8

Formule I  Y = H ; X = m-$CF_3$ ; $R_1$ = $R_2$ = $CH_3$

Cristaux F = 158 °C (isopropanol) ; rendement 35 %

Exemple 7

(Trifluorométhyl-3 phényl)-1 dihydro-1,2 oxo-2 méthyl-3 isopropyl-7 naphtyridine-1,8

Formule I $Y = H$ ; $X = m\text{-}CF_3$ ; $R_1$ = isopropyle ; $R_2 = CH_3$

Cristaux F = 115 °C (hexane) ; rendement 30 %

Exemple 8

(Trifluorométhyl-3 phényl)-1 dihydro-1,2 oxo-2 méthyl-3 cyclopropyl-7 naphtyridine-1,8

Formule I $Y = H$ ; $X = m\text{-}CF_3$ ; $R_1$ = cyclopropyle ; $R_2 = CH_3$

Cristaux F = 147 °C (isopropanol) ; rendement 32 %

Exemple 9

(Méthyl-2 chloro-3 phényl)-1 dihydro-1,2 oxo-2 méthyl-3 naphtyridine-1,8

Formule I $X = o\text{-}CH_3$ ; $Y = m\text{-}Cl$ ; $R_1 = H$ ; $R_2 = CH_3$

Cristaux F = 192 °C (isopropanol) ; rendement 25 %

Exemple 10

(Trifluorométhyl-3 phényl)-1 dihydro-1,2 oxo-2 méthyl-3 naphtyridine-1,8

Formule I $R_1 = Y = H$ ; $X = m\text{-}CF_3$ ; $R_2 = CH_3$

On porte au reflux durant 1 h 15 min un mélange de 20 g de (trifluorométhyl-3 phénylamino)-2 nicotinaldéhyde synthétisés à l'exemple 2, de 10 g de propionate de sodium et de 20 ml d'anhydride propionique.

Le mélange réactionnel est ensuite refroidi, additionné de 100 ml d'eau et agité 30 min à température ambiante. On extrait au dichlorométhane, la phase organique est lavée à l'eau, avec une solution de soude 10 % puis encore à l'eau, séchée sur sulfate de sodium et concentrée sous vide. Le résidu obtenu cristallise dans le pentane, les cristaux sont essorés, lavés à l'éther isopropylique et séchés. Les 17,6 g de cristaux obtenus sont recristallisés dans l'isopropanol conduisant à 14,3 g de (trifluorométhyl-3 phényl)-1 dihydro-1,2 oxo-2 méthyl-3 naphtyridine-1,8 sous forme de cristaux de point de fusion 189-190 °C identiques à ceux décrits à l'exemple 3.

Selon cette méthode, est préparé le dérivé suivant :

Exemple 11

Phényl-1 dihydro-1,2 oxo-2 méthyl-3 naphtyridine-1,8

Formule I $R_1 = X = Y = H$ ; $R_2 = CH_3$

Cristaux F = 217-8 °C (éthanol) ; rendement 56 %

On a préparé selon un procédé analogue les composés suivants :
(nitro-3 phényl)amino-2 hydroxy méthyl-3 pyridine

Formule III $R_1 = Y = H$ ; $X = m\text{-}NO_2$

Cristaux F = 150-5 °C ; rendement : 84 %

(nitro-3 phényl amino)-2 nicotinaldéhyde

Formule II $R_1 = Y = H$ ; $X = m\text{-}NO_2$

Cristaux F = 160-2 °C (acétonitrile) ; rendement : 70 %

Exemple 12

On a reproduit le procédé de l'exemple 10 pour préparer le : (nitro-3 phényl)-1 dihydro-1,2 oxo-2 méthyl-3 naphtyridine-1,8

Formule I $R_1 = Y = H$ ; $X = m\text{-}NO_2$ ; $R_2 = CH_3$

Cristaux F = 231-2 °C (acétonitrile) ; rendement : 52 %

Exemples 13 et 14

En opérant comme dans les exemples précédents on a préparé les composés suivants : (méthoxy-3 phényl) amino-2 hydroxy méthyl-3 pyridine

Formule III $R_1 = Y = H$ ; $X = m\text{-}OCH_3$

Cristaux F = 94-95 °C ; rendement : 98 %

(méthyl-4 phényl) amino-2 hydroxy méthyl-3 pyridine

Formule III $R_1 = Y = H$ ; $X = p\text{-}CH_3$

Huile épaisse ; rendement : 98 %

(méthoxy-3 phényl amino)-2 nicotinaldéhyde

Formule II $R_1 = Y = H$ ; $X = m\text{-}OCH_3$

Cristaux F = 65-66 °C (éther isopropylique) ; rendement : 75 %

(méthyl-4 phényl amino)-2 nicotinaldéhyde

Formule II $R_1 = Y = H$ ; $X = p\text{-}CH_3$

Cristaux F = 55-8 °C (éther isopropylique) ; rendement 72 %

Ce qui a permis, en mettant en œuvre l'exemple 10, de préparer les composés suivants :

Exemple 13

(méthoxy-3 phényl)-1 dihydro-1,2 oxo-2 méthyl-3 naphtyridine-1,8

Formule I $R_1 = Y = H$ ; $X = m\text{-}OCH_3$ ; $R_2 = CH_3$

Cristaux F = 196-197 °C (isopropanol) ; rendement 40 %

Exemple 14

(méthyl-4 phényl)-1 dihydro-1,2 oxo-2 méthyl-3 naphtyridine-1,8

Formule I $R_1 = Y = H$ ; $X = p\text{-}CH_3$ ; $R_2 = CH_3$

Cristaux F = 212-213 °C (isopropanol) ; rendement 39 %.

Les propriétés pharmacologiques des produits selon l'invention ont été étudiées.

Activité anti-ulcères

Méthode

Des lots de 10 à 30 rats mâles de souche OFA (Iffa Credo), pesant 160-180 g, sont mis à la diète hydrique 24 h avant l'administration orale du produit étudié. Trente minutes plus tard, un agent ulcérigène médicamenteux ou un agent nécrosant (exemple : éthanol absolu) est administré par voie orale à des doses qui entraînent chez les animaux témoins une ulcération gastrique maximale. Les estomacs sont prélevés, selon le test, respectivement 6 et 1 h après le dernier traitement. Les lésions gastriques sont alors évaluées macroscopiquement (cotation et mesure de l'ampleur des ulcères).

Résultats

Ils sont exprimés sous forme de % d'inhibition des ulcères. La dose active 50 est déterminée graphiquement.

| Dose mg.kg-1 VO | % de protection des lésions provoquées | | | | | |
|---|---|---|---|---|---|---|
| | (1) administration d'un agent·médicamenteux ulcérigène | | | (2) administration d'un agent nécrosant | | |
| | Ex. 3 et 10 | Ex. 4 | Ex. 11 | Ex. 3 et 10 | Ex. 4 | Ex. 11 |
| 0,06 | | | | 0 | | 86 |
| 0,125 | | | | 17 | | |
| 0,25 | | | 1 | 42 | | 95 |
| 0,5 | | | 34 | 64 | | |
| 1 | | | 75 | 79 | 6 | 94 |
| 2 | 15 | | 100 | | 52 | |
| 4 | 22 | 11 | | | 87 | 98 |
| 8 | 62 | 40 | | | | |
| 16 | 87 | 68 | | | | |
| 32 | 95 | 81 | | | | |
| 64 | | | | | | |
| 256 | | | | | | |
| DA 50 mg.kg-1 VO | 6,5 | 12 | 0,67 | 0,37 | 2,2 | < 0,06 |

(1) agent anti-inflammatoire non stéroïdien
(2) éthanol

## Activité anti-sécrétoire

Méthode

Une ligature de pylore est pratiquée sous anesthésie à l'éther à des lots de 5 rats mâles de souche OFA (Iffa Credo) pesant 180-200 g. Le produit étudié est administré par voie orale ou sous-cutanée au temps de la ligature. Les animaux sont sacrifiés 4 heures plus tard et l'acidité du liquide gastrique recueilli est titrée par la soude 0,1 N à pH 4 et 7.

Résultats

Ils sont exprimés en pourcentage d'inhibition de la sécrétion acide totale. La dose active 50 est déterminée à partir de la régression linéaire. La signification est donnée par le test « t » de la régression linéaire. La signification est donnée par le test « t » de Student (* et ** pour $p < 0,05$ et $p < 0,01$).

(Voir Tableau page 9.)

| Dose mg.kg-1 VO | % d'inhibition de la sécrétion acide totale | | |
|---|---|---|---|
| | Administrés VO | Administrés SC | |
| | Exemples 3 et 10 | Exemples 3 et 10 | Exemple 11 |
| 0,03 | | | 37 |
| 0,06 | | | 46 |
| 0,125 | 2 | | 63* |
| 0,25 | 18 | 4 | 76** |
| 0,5 | 59* | 38 | 81** |
| 1 | 56 | 65** | |
| 2 | 75** | 90** | |
| DA50 mg.kg-1 VO | 0,70 (0,34-1,44) | 0,72 (0,44-1,18) | 0,065 (0,032-0,135) |

Activité anti-inflammatoire

Méthode

Des lots de 6 à 12 rats mâles de souche CD (Charles River), pesant 120-130 g, reçoivent pour nourriture et boisson une solution aqueuse à 9 ‰ de chlorure de sodium et 200 ‰ de glucose 18 heures avant le test.

Le produit étudié est administré par voie orale en deux fois : une demi-dose 2 heures et une demi-dose 30 minutes avant injection sous-cutanée dans le coussinet plantaire d'une patte postérieure de 0,05 ml d'une solution aqueuse de carragénine à 1,5 %. Le volume de la patte est mesuré par pléthysmographie à 1,5 %. Le volume de la patte est mesuré par pléthysmographie à intervalles réguliers.

Résultats

Le tableau donne les pourcentages d'inhibition de l'œdème au maximum de son amplitude chez les témoins. La dose active 50 et ses limites sont déterminées à partir de la régression linéaire. La signification des résultats est donnée par le test « t » de Student (*, ** et *** pour $p < 0,05$, $p < 0,01$ et $p < 0,001$).

| Dose mg.kg-1 VO | % d'inhibition de l'oedème | |
|---|---|---|
| | Exemples 3 et 10 | Exemple 4 |
| 4 | 10 | 14 |
| 16 | 31* | 21 |
| 64 | 47*** | 80*** |
| 128 | 61*** | 70*** |
| 256 | 64*** | 83*** |
| DA50 mg.kg-1 VO | 73,3 (47,6 - 112,9) | non calculable |

## Activité analgésique

Méthode

Des lots de 6 à 18 souris mâles de souche CD1 (Charles River), pesant 18-22 g, reçoivent par voie intrapéritonéale, une solution hydro-alcoolique de phénylbenzoquinone à 0,02 % 1 heure après traitement par voie orale.

Le nombre des réactions douloureuses est compté de la 5$^{e}$ semaine à la 10$^{e}$ minute.

Résultats

Le tableau ci-après donne le pourcentage d'inhibition des torsions et étirements. La dose active et ses limites sont déterminées à partir de la régression linéaire.

| Dose mg.kg-1 VO | % d'inhibition des réactions douloureuses | |
|---|---|---|
| | Exemples 3 et 10 | Exemple 4 |
| 8 | 6 | |
| 16 | 30 ** | 6 |
| 32 | 26 * | 39 * |
| 64 | 57 *** | 37 * |
| 128 | 58 *** | 79 *** |
| 256 | 73 *** | 93 *** |
| DA50 mg.kg-1 VO | 69,4 (45,7 - 105,5) | 62,0 (46,8 - 82,0) |

## Toxicologie

Les études toxicologiques réalisées chez le rat à jeun Sprague Dawley après administration par voie orale ont permis de montrer que les produits exemplifiés possèdent une dose létale 50 % supérieure (produits des exemples 3, 10 et 4) ou égale (produit de l'exemple 11) à 256 mg.kg$^{-1}$.

Il apparaît en conclusion que les produits selon l'invention et leurs sels d'addition non toxiques pourront avantageusement être utilisés comme principes actifs dans des médicaments utilisables par voie orale ou injectable. Lesdits médicaments contiendront de 200 à 600 mg de principe actif et seront utilisables pour le traitement des ulcères gastro-intestinaux.

Tableau I

5145-01

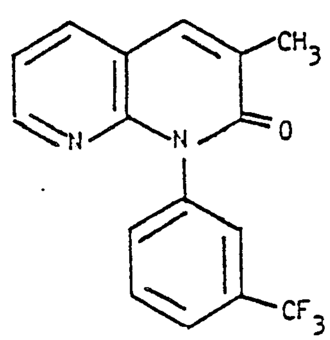

Exemples 3 et 10

(Suite page 11)

5145-02

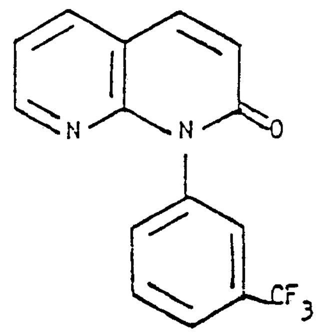

Exemple 4

5145-11

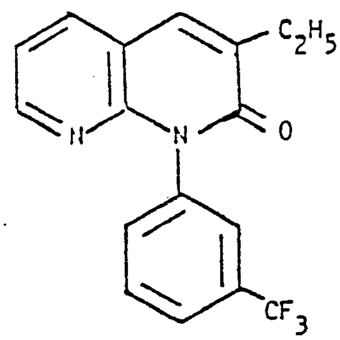

Exemple 5

5145-05

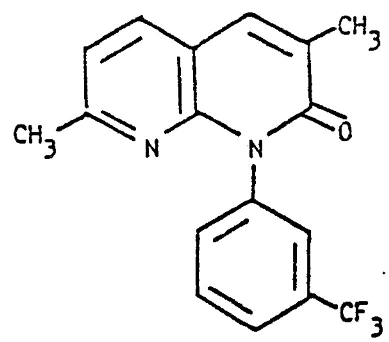

Exemple 6

5145-07

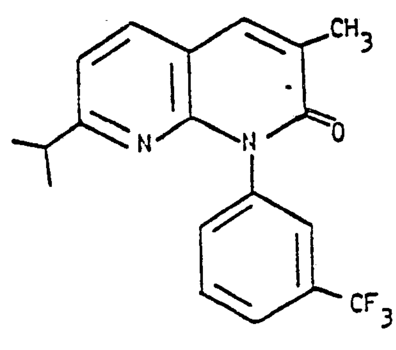

Exemple 7

5145-08

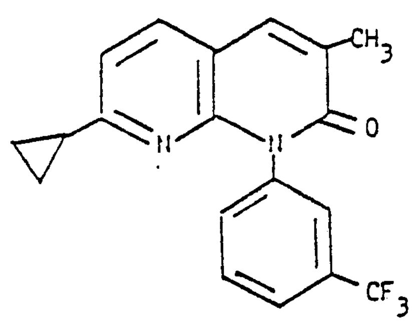

Exemple 8

5145-04

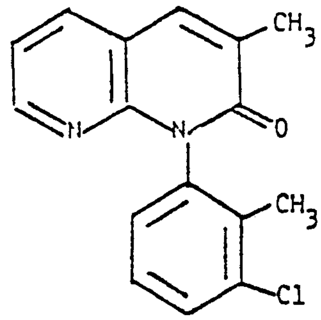

Exemple 9

5145-12

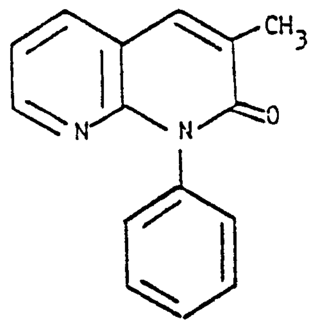

Exemple 11

5145-14

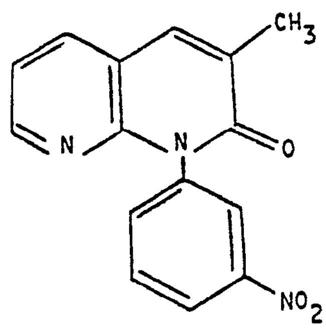

Exemple 12

**Revendications** (pour les Etats contractants : BE, CH, DE, FR, GB, IT, LI, LU, NL, SE)

1. Phényl-1-naphtyridines-1,8 caractérisées en ce qu'elles répondent à la formule (I) :

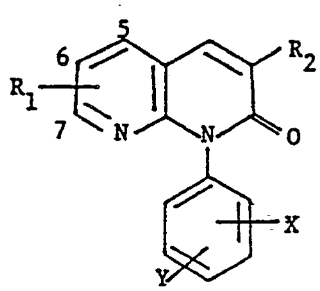

(I)

dans laquelle :

$R_1$ représente un atome d'hydrogène, un radical méthyle, isopropyle ou cyclopropyle, et peut être en position 5, 6 ou 7 de la naphtyridine,

$R_2$ représente un atome d'hydrogène, un radical alkyle inférieur linéaire ou ramifié de 1 à 5 atomes de carbone,

X et/ou Y représentent un atome d'hydrogène, un radical alkyle inférieur de 1 à 5 atomes de carbone, un atome d'halogène, un groupement trifluorométhyle, méthoxy, nitro, thiométhyle et peuvent être en position ortho, méta ou para.

2. Phényl-1-naphtyridines-1,8 selon la revendication 1, caractérisées en ce que $R_2 = H$, $CH_3$ ou $C_2H_5$.

3. Phényl-1-naphtyridines-1,8 selon la revendication 1 ou 2, caractérisées en ce que $R_1$ se trouve en position 7 de la naphtyridine.

4. Phényl-1-naphtyridine-1,8 caractérisée en ce qu'elle est choisie parmi les composés suivants :
— phényl-1 dihydro-1,2 oxo-2 méthyl-3 naphtyridine-1,8 ;
— (trifluorométhyl-3 phényl)-1 dihydro-1,2 oxo-2 naphtyridine-1,8 ;
— (trifluorométhyl-3 phényl)-1 dihydro-1,2 oxo-2 méthyl-3 naphtyridine-1,8 ; et
— (trifluorométhyl-3 phényl)-1 dihydro-1,2 oxo-2 éthyl-3 naphtyridine-1,8.

5. Procédé de préparation des phényl-1-naphtyridines-1,8 de la formule (I) définie à l'une des revendications 1 à 4, caractérisé en ce qu'on fait réagir un aldéhyde de formule (II) :

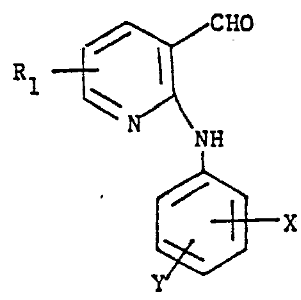

(II)

dans laquelle $R_1$, X et Y sont tels que définis aux revendications 1 à 4, sur un anhydride de formule V :

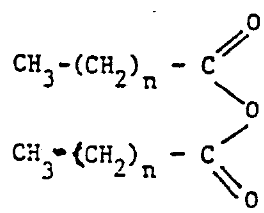

(V)

dans laquelle n est un nombre de 0 à 4 ou sur un sel de sodium de l'acide correspondant.

6. Procédé selon la revendication 5, caractérisé en ce qu'on réalise la réaction dans un solvant à une température de 100 à 200 °C environ.

7. Procédé selon la revendication 6, caractérisé en ce que le solvant est la méthylpyrrolidone.

8. Composition thérapeutique caractérisée en ce qu'elle renferme, en association avec un excipient physiologiquement acceptable, au moins un composé de formule (I) selon l'une quelconque des revendications 1 à 4.

9. Composition thérapeutique utile pour son activité anti-ulcères, anti-inflammatoire et analgésique, caractérisée en ce qu'elle renferme en association avec un excipient physiologiquement acceptable, au moins un composé de formule (I), selon l'une quelconque des revendications 1 à 4.

**Revendications** (pour l'Etat contractant AT)

1. Procédé de préparation de phényl-1-naphtyridines-1,8 caractérisées en ce qu'elles répondent à la formule (I) :

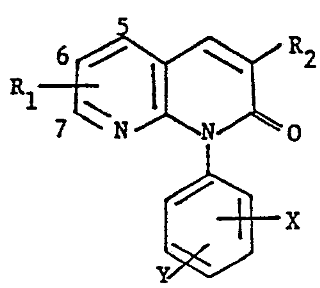

(I)

dans laquelle :
— $R_1$ représente un atome d'hydrogène, un radical méthyle, isopropyle ou cyclopropyle, et peut être en position 5, 6 ou 7 de la naphtyridine,

— $R_2$ représente un atome d'hydrogène, un radical alkyle inférieur linéaire ou ramifié de 1 à 5 atomes de carbone,

— X et/ou Y représentent un atome d'hydrogène, un radical alkyle inférieur de 1 à 5 atomes de carbone, un atome d'halogène, un groupement trifluorométhyle, méthoxy, nitro, thiométhyle et peuvent être en position ortho, méta ou para ;

et en ce que l'on fait réagir un aldéhyde de formule (II) :

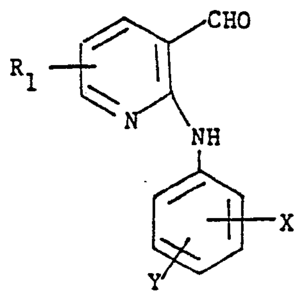

(II)

dans laquelle $R_1$, X et Y sont tels que définis ci-dessus avec un anhydride de formule V :

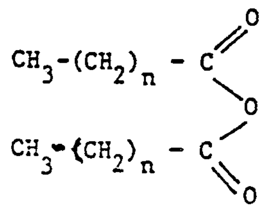

(V)

dans laquelle n est un nombre de 0 à 4 ou sur un sel de sodium de l'acide correspondant, selon la réaction connue de Perkin.

2. Procédé selon la revendication 1, caractérisé en ce qu'on réalise la réaction dans un solvant à une température de 100 à 200 °C environ.

3. Procédé selon la revendication 2, caractérisé en ce que le solvant est la méthylpyrrolidone.

4. Procédé de préparation d'une composition thérapeutique, caractérisé en ce qu'on réalise le mélange d'au moins un composé de formule (I) telle que définie à la revendication 1, avec un excipient physiologiquement acceptable.

5. Procédé de préparation d'une composition thérapeutique, utile pour son activité anti-ulcères, anti-inflammatoire et analgésique, caractérisé en ce qu'on réalise le mélange d'au moins un composé de formule (I) telle que définie à la revendication 1, avec un excipient physiologiquement acceptable.

6. Procédé selon la revendication 4 ou 5, caractérisé en ce que dans la formule (I) précitée $R_2 = H$, $CH_3$ ou $C_2H_5$.

7. Procédé selon l'une des revendications 4 ou 5, caractérisé en ce que dans la formule (I) précitée $R_1 = H$, ou un isopropyle ou cyclopropyle en position 7 de la naphtyridine.

8. Procédé selon l'une des revendications 4 à 5, caractérisé en ce que le composé de formule (I) précitée est choisi parmi les composés suivants :

— phényl-1 dihydro-1,2 oxo-2 méthyl-3 naphtyridine-1,8 ;

— (trifluorométhyl-3 phényl)-1 dihydro-1,2 oxo-2 naphtyridine-1,8 ;

— (trifluorométhyl-3 phényl)-1 dihydro-1,2 oxo-2 méthyl-3 naphtyridine-1,8 ; et

— (trifluorométhyl-3 phényl)-1 dihydro-1,2 oxo-2 éthyl-3 naphtyridine-1,8.

**Claims** (for Contracting States : BE, CH, DE, FR, GB, IT, LT, LU, NL, SE)

1. 1-phenyl 1,8-naphthyridines, characterized in that they correspond to formula (I) :

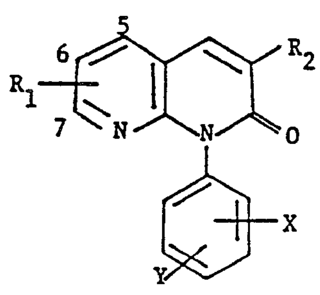

(I)

in which :

— $R_1$ represents a hydrogen atom, a methyl, isopropyl or cyclopropyl radical and may be in 5, 6 or 7 position of the naphthyridine ;

— $R_2$ represents a hydrogen atom, a straight or branched lower alkyl radical of 1 to 5 carbon atoms,

— X and/or Y represent a hydrogen atom, a lower alkyl radical of 1 to 5 carbon atoms, a halogen atom, a trifluoromethyl, methoxy, nitro, thiomethyl group and may be in ortho, meta or para position.

2. 1-phenyl 1,8-naphthyridines according to claim 1, characterized in that $R_2$ = H, $CH_3$ or $C_2H_5$.

3. 1-phenyl 1,8-naphthyridines according to claim 1 or 2, characterized in that $R_1$ is in 7 position of naphthyridine.

4. 1-phenyl 1,8-naphthyridine, characterized in that it is selected among the following compounds :

— 1-phenyl 1,2-dihydro 2-oxo 3-methyl 1,8-naphthyridine ;

— 1-(3-trifluoromethyl phenyl) 1,2-dihydro 2-oxo 1,8-naphthyridine ;

— 1-(3-trifluoromethyl phenyl) 1,2-dihydro 2-oxo 3-methyl 1,8-naphthyridine ; and

— 1-(3-trifluoromethyl phenyl) 1,2-dihydro 2-oxo 3-ethyl 1,8-naphthyridine.

5. A process for the preparation of 1-phenyl 1,8-naphthyridines of formula (I) defined in one of claims 1 to 4, characterized in that an aldehyde of formula (II) :

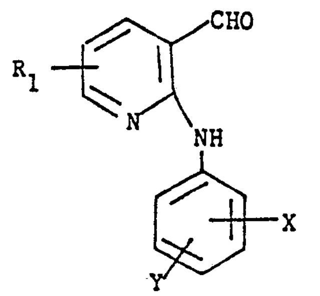

(II)

is reacted, in which $R_1$, X and Y are as defined in claims 1 to 4, on an anhydride of formula V :

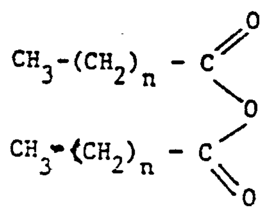

(V)

in which n is an integer of 0 to 4, or on a sodium salt of the corresponding acid.

6. A process according to claim 5, characterized in that the reaction is carried out in a solvent at a temperature of about 100 to 200 °C.

7. A process according to claim 6, characterized in that the solvent is methylpyrrolidone.

8. A therapeutical composition, characterized in that it includes, in association with a physiologically acceptable excipient, at least one compound of formula (I) according to any one of claims 1 to 4.

9. A therapeutical composition useful for its anti-ulcer, anti-inflammatory and analgesic activity, characterized in that it includes, in association with a physiologically acceptable excipient, at least one compound of formula (I) according to any one of claims 1 to 4.

**Claims** (for Contracting State AT)

1. A process for the preparation of 1-phenyl 1,8-naphthyridines, characterized in that they correspond to formula (I) :

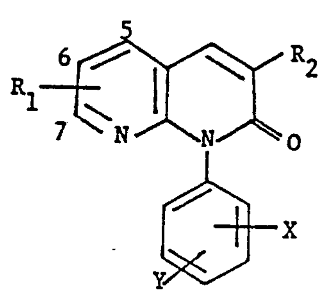

(I)

in which

— $R_1$ represents a hydrogen atom, a methyl, isopropyl or cyclopropyl radical and may be in 5, 6 or 7 position of the naphthyridine,

— $R_2$ represents a hydrogen atom, a straight or branched lower alkyl radical of 1 to 5 carbon atoms,

X and/or Y represent a hydrogen atom, a lower alkyl radical of 1 to 5 carbon atoms, a halogen atom, a trifluoromethyl, methoxy, nitro, thiomethyl group and may be in ortho, meta or para position ; and in that an aldehyde of formula (II) :

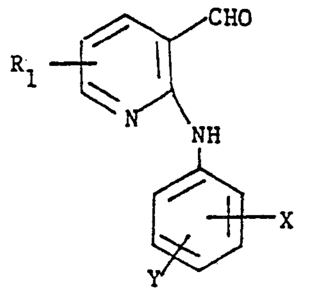

(II)

is reacted, in which $R_1$, X and Y are as defined hereinabove, with an anhydride of formula V :

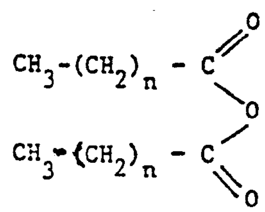

(V)

in which n is an integer of 0 to 4, or on a sodium salt of the corresponding acid, according to the known reaction of Perkin.

2. A process according to claim 1, characterized in that the reaction is carried out in a solvent at a temperature of about 100 to 200 °C.

3. A process according to claim 2, characterized in that the solvent is methylpyrrolidone.

4. A process for the preparation of a therapeutical composition, characterized in that a mixture is produced of at least one compound of formula (I) such as defined in claim 1, with a physiologically acceptable excipient.

5. A process for the preparation of a therapeutical composition useful for its anti-ulcer, anti-inflammatory and analgesic activity, characterized in that a mixture is produced of at least one compound of formula (I), such as defined in claim 1, with a physiologically acceptable excipient.

6. A process according to claim 4 or 5, characterized in that in the previously cited formula (I) $R_2 = H$, $CH_3$ or $C_2H_5$.

7. A process according to one of claims 4 or 5, characterized in that in the previously cited formula (I) $R_1 = H$ or an isopropyl or cyclopropyl in 7 position of the naphthyridine.

8. A process according to one of claims 4 to 5, characterized in that the previously cited compound of formula (I) is selected among the following compounds :

— 1-phenyl 1,2-dihydro 2-oxo 3-methyl 1,8-naphthyridine ;

— 1-(3-trifluoromethyl phenyl) 1,2-dihydro 2-oxo 1,8-naphthyridine ;

— 1-(3-trifluoromethyl phenyl) 1,2-dihydro 2-oxo 3-methyl 1,8-naphthyridine ; and

— 1-(3-trifluoromethyl phenyl) 1,2-dihydro 2-oxo 3-ethyl 1,8-naphthyridine.

**Patentansprüche** (für die Vertragsstaaten : BE, CH, DE, FR, GB, IT, LI, LU, NL, SE)

1. 1-Phenyl-1,8-naphthyridine, dadurch gekennzeichnet, daß sie der Formel (I) entsprechen :

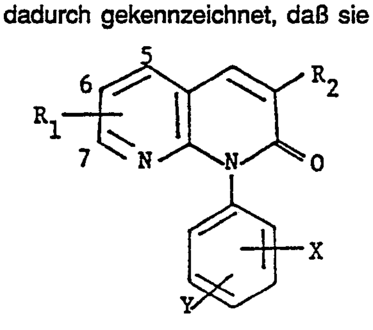

(I)

worin
— $R_1$ ein Wasserstoffatom, eine Methyl-, Isopropyl- oder Cyclopropylgruppe darstellt und in Position 5, 6 oder 7 des Naphthyridins stehen kann,
— $R_2$ ein Wasserstoffatom, eine gerade oder verzweigte Niedrigalkylgruppe mit 1 bis 5 Kohlenstoffatomen darstellt,
— X und/oder Y ein Wasserstoffatom, eine Niedrigalkylgruppe mit 1 bis 5 Kohlenstoffatomen, ein Halogenatom, eine Trifluormethyl-, Methoxy-, Nitro-, Thiomethylgruppe darstellen und in der Position ortho, meta oder para stehen können.

2. 1-Phenyl-1,8-naphthyridine nach Anspruch 1, dadurch gekennzeichnet, daß $R_2 = H$, $Ch_3$ oder $C_2H_5$.

3. 1-Phenyl-1,8-naphthyridine nach Anspruch 1 oder 2, dadurch gekennzeichnet, daß sich $R_1$ in Position 7 des Naphthyridins befindet.

4. 1-Phenyl-1,8-naphthyridin, dadurch gekennzeichnet, daß es ausgewählt ist aus den folgenden Verbindungen :
— 1-Phenyl-1,2-dihydro-2-oxo-3-methyl-1,8-naphthyridin ;
— 1-(3-Trifluormethylphenyl)-1,2-dihydro-2-oxo-1,8-naphthyridin ;
— 1-(3-Trifluormethylphenyl)-1,2-dihydro-2-oxo-3-methyl-1,8-naphthyridin ; und
— 1-(3-Trifluormethylphenyl)-1,2-dihydro-2-oxo-3-ethyl-1,8-naphthyridin.

5. Verfahren zur Herstellung der 1-Phenyl-1,8-naphthyridine der Formel (I) nach einem der Ansprüche 1 bis 4, dadurch gekennzeichnet, daß man ein Aldehyd der Formel (II) :

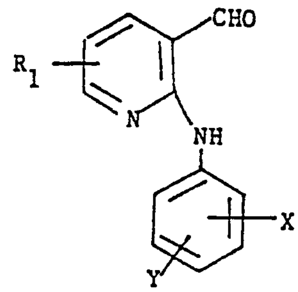

(II)

worin $R_1$, X und Y wie in den Ansprüchen 1 bis 4 definiert sind, mit einem Anhydrid der Formel V :

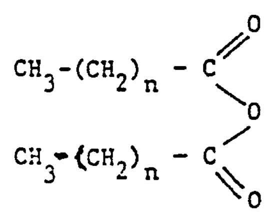

(V)

worin n eine Zahl von 0 bis 4 bedeutet, oder mit einem Natriumsalz der entsprechenden Säure reagieren läßt.

6. Verfahren nach Anspruch 5, dadurch gekennzeichnet, daß man die Umsetzung in einem Lösungsmittel bei einer Temperatur von etwa 100 bis 200 °C durchführt.

7. Verfahren nach Anspruch 6, dadurch gekennzeichnet, daß das Lösungsmittel Methylpyrrolidon ist.

8. Therapeutische Zusammensetzung, dadurch gekennzeichnet, daß sie in Kombination mit einem physiologisch akzeptablen Exzipienten mindestens eine Verbindung der Formel (I) nach einem der Ansprüche 1 bis 4 enthält.

9. Therapeutische Zusammensetzung, die wegen ihrer geschwürhemmenden, entzündungshemmenden und analgetischen Aktivität nützlich ist, dadurch gekennzeichnet, daß sie in Kombination mit einem physiologisch akzeptablen Exzipienten mindestens eine Verbindung der Formel (I) nach einem der Ansprüche 1 bis 4 enthält.

**Patentansprüche** (für den Vertragsstaat AT)

1. Verfahren zur Herstellung von 1-Phenyl-1,8-naphthyridinen, dadurch gekennzeichnet, daß sie der Formel (I) entsprechen :

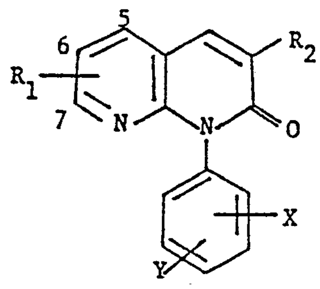

(I)

worin :

— $R_1$ ein Wasserstoffatom, eine Methyl-, Isopropyl- oder Cyclopropylgruppe darstellt und in Position 5, 6 oder 7 des Naphthyridins stehen kann,

— $R_2$ ein Wasserstoffatom, eine gerade oder verzweigte Niedrigalkylgruppe mit 1 bis 5 Kohlenstoffatomen darstellt,

— X und/oder Y ein Wasserstoffatom, eine Niedrigalkylgruppe mit 1 bis 5 Kohlenstoffatomen, ein Halogenatom, eine Trifluormethyl-, Methoxy-, Nitro-, Thiomethylgruppe darstellen und in der Position ortho, meta oder para stehen können ; und dadurch gekennzeichnet, daß man ein Aldehyd der Formel (II) ;

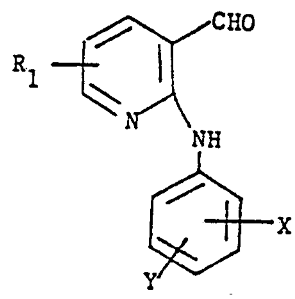

(II)

worin $R_1$, X und Y wie oben definiert sind, mit einem Anhydrid der Formel V :

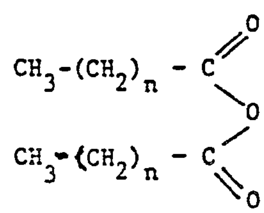

(V)

worin n eine Zahl von 0 bis 4 bedeutet, oder mit einem Natriumsalz der entsprechenden Säure gemäß der bekannten Perkinschen Reaktion zur Umsetzung bringt.

2. Verfahren nach Anspruch 1, dadurch gekennzeichnet, daß man die Umsetzung in einem Lösungsmittel bei einer Temperatur von etwa 100 bis 200 °C durchführt.

3. Verfahren nach Anspruch 2, dadurch gekennzeichnet, daß das Lösungsmittel Methylpyrrolidon ist.

4. Verfahren zur Herstellung einer therapeutischen Zusammensetzung, dadurch gekennzeichnet, daß man die Mischung mindestens einer Verbindung der Formel (I) wie in Anspruch 1 definiert mit einem physiologisch akzeptablen Exzipienten vornimmt.

5. Verfahren zur Herstellung einer therapeutischen Zusammensetzung, die wegen ihrer geschwürhemmenden, entzündungshemmenden und analgetischen Aktivität nützlich ist, dadurch gekennzeichnet, daß man die Mischung mindestens einer Verbindung der Formel (I) wie in Anspruch 1 definiert mit einem physiologisch akzeptablen Exzipienten vornimmt.

6. Verfahren nach Anspruch 4 oder 5, dadurch gekennzeichnet, daß in der genannten Formel (I) $R_2 = H$, $CH_3$ oder $C_2H_5$.

7. Verfahren nach einem der Ansprüche 4 oder 5, dadurch gekennzeichnet, daß in der genannten Formel (I) $R_1$ H oder ein Isopropyl oder Cyclopropyl in Position 7 des Naphthyridins ist.

8. Verfahren nach einem der Ansprüche 4 bis 5, dadurch gekennzeichnet, daß die Verbindung der Formel (I) ausgewählt ist aus den folgenden Verbindungen :

— 1-Phenyl-1,2-dihydro-2-oxo-3-methyl-1,8-naphthyridin ;

— 1-(3-Trifluormethylphenyl)-1,2-dihydro-2-oxo-1,8-naphthyridin ;

— 1-(3-Trifluormethylphenyl)-1,2-dihydro-2-oxo-3-methyl-1,8-naphthyridin ; und

— 1-(3-Trifluormethylphenyl)-1,2-dihydro-2-oxo-3-ethyl-1,8-naphthyridin.